# FASCICULE DE BREVET EUROPEEN

(11) **EP 0 453 368 B1**
(45) Date de publication et mention de la délivrance du brevet: **31.07.1996**
(21) Numéro de dépôt: 91401031.9
(22) Date de dépôt: 18.04.1991
(51) Int. Cl.: C12P 7/24

(54) **Production de vanilline par bioconversion de précurseurs benzéniques**
Herstellung von Vanillin durch biologische Umwandlung von Benzolvorstufen
Production of vanillin by bioconversion of benzenic precursors

(30) Priorité: 19.04.1990 FR 9005003
(43) Date de publication de la demande: 23.10.1991
(73) Titulaire: PERNOD-RICARD, F-75008 Paris (FR)
(72) Inventeur: Gross, Brigitte, Galliéni, F-78220 Viroflay (FR); Asther, Marcel, F-78310 Maurepas (FR); Corrieu, Georges, F-78220 Viroflay (FR); Brunerie, Pascal, F-94100 Saint Maur (FR)
(74) Mandataire: Warcoin, Jacques

(56) Documents cités:
- EP-A- 405 197
- CH-A- 667 673
- JOURNAL OF THE SCIENCE OF FOOD & AGRICULTURE, vol. 36, no. 10, 1985, Barking (GB); F. AGOSIN et al., pp. 925-935/
- AGRICULTURAL & BIOLOGICAL CHEMISTRY, vol. 41, no. 6, 1977, Tokyo (JP); K. TASADA, pp. 925-929/

## Description

La présente invention concerne un procédé de production de vanilline par bioconversion de précurseurs benzéniques.

La vanilline est obtenue actuellement soit par synthèse chimique, soit à l'état naturel par extraction à partir de gousse de vanille.

Le but de la présente invention est d'obtenir un procédé de production de vanilline simple à industrialiser et qui permette d'obtenir une vanilline qui puisse être considérée par les règlementations en vigueur, notamment dans les pays de la CEE comme naturelle. Tel est le cas des produits obtenus par bioconversion de précurseurs naturels à l'aide de microorganismes.

L'article de Agric. Biol. Chem. 41(6), 925-929 (1977) décrit la dégradation de l'eugénol en acide protocatechuique par une bactérie au cours de laquelle est obtenue la vanilline comme métabolite intermédiaire. L'article de J. Sci. Food. Agric. Vol. 36 10(985) 925-935 mentionne la dégradation de la lignine comprenant un ester d'acide férulique par Picnoporus Cinnabarinus sans mention de production de vanilline.

Pour ce faire, la présente invention a pour objet un procédé de production de vanilline caractérisé en ce qu'on effectue une culture de champignon Basidiomycète du genre Pycnoporus ou de leurs variants et mutants dans un milieu de culture dans lequel un précurseur benzènique de la vanilline a été ajouté et en ce que l'on récupère la vanilline produite après bioconversion dudit précurseur, la souche de Pycnoporus étant sélectionnée pour sa propriété métabolique de dégrader lesdits précurseurs avec accumulation de vanilline dans le milieu.

A titre de précurseur benzénique, on utilisera en particulier des dérivés en position 1 du 4-hydroxy-3-méthoxy-benzène, tel que l'acide vanillique ou l'acide férulique.

De façon particulièrement appropriée, on utilisera le champignon de l'espèce Pycnoporus cinnabarinus, notamment les souches CNCM N° 1-937 et 1-938 ou leurs variants et mutants qui possèdent la propriété métabolique de dégrader les précurseurs benzéniques de la vanilline, tels que l'acide férulique avec accumulation de vanilline dans le milieu, à un degré élevé.

Avantageusement, on utilisera des précurseurs provenant de substrats végétaux naturels, de préférence riches en dits précurseurs. On peut mentionner l'acide férulique présent dans les membranes cellulaires de nombreux monocotylédones et dicotylédones, telles que par exemple les cossettes de betteraves, sous produit de l'industrie sucrière, ou des grains d'aleurone du blé ou de différentes céréales. Les sources naturelles d'acide férulique sont donc relativement abondantes. Un autre précurseur benzénique d'origine naturelle utilisable selon l'invention est l'isoeugenol.

D'autre part, des composés comme l'acide parahydroxy-benzoïque, le parahydroxy-benzaldéhyde, l'alcool vanillique et les stilbènes entre autres, peuvent être utilisés.

Dans un mode de mise en oeuvre préférentielle du procédé selon l'invention, le champignon est tout d'abord cultivé en milieu liquide agité, et l'on n'ajoute le précurseur qu'après qu'une biomasse importante possédant les capacités enzymatiques optimum pour la bioconversion dudit précurseur soit obtenue.

Ainsi avantageusement, dans le cas de la culture du champignon Pycnoporus cinnabarinus, le précurseur est ajouté lorsque la biomasse nécessaire est obtenue soit 3 jours dans les conditions de culture du milieu de l'annexe 2 décrit de façon détaillée ci-après.

Lorsqu'on ajoute le précurseur en début de culture, la bioconversion en vanilline est observée mais avec des rendements beaucoup plus faibles.

Dans un mode de réalisation particulier du procédé selon l'invention, l'inoculum par lequel le champignon est inoculé dans le milieu de culture consiste en des fragments de mycelium ou des spores, ou des précultures de ces inocullum.

On pourra introduire une quantité de précurseurs allant jusqu'à 500g/ml par milieu de culture.

Dans le procédé selon l'invention, on utilisera au moins 15 fragments de mycelium pour 100 ml de milieu de culture et 10⁵ à 10⁷ spores/ml de milieu. Le pH du milieu de culture peut être compris entre 2,5 et 6,5. La température d'incubation des cultures peut être comprise entre 25 et 40°C.

De façon avantageuse, on récupère la vanilline lorsque le précurseur n'est plus détectable dans le milieu culture et avant sa bioconversion en alcool vanillique.

Pour récupérer la vanilline, on peut selon des procédés connus de l'homme de l'art, l'extraire des milieux de culture avec des solvants suivie d'une distillation, on peut aussi procéder à une cristallisation fractionnée ou à une chromatographie préparative industrielle directement à partir des milieux de culture.

Selon le procédé selon l'invention, le précurseur peut être apporté séquentiellement ou en continu et la vanilline peut être récupérée également séquentiellement ou en continu.

Dans les conditions de culture du milieu de l'annexe 2 décrit de façon détaillée ci-après, on récupère la vanilline 4 ou 5 jours après l'apport en précurseur.

D'autres caractéristiques et avantages de la présente invention apparaîtront à la lumière de la description détaillée qui va suivre.

La description fait référence aux figures 1 à 5.
- La figure 1 :: représente la bioconversion de l'acide vanillique ajouté lors de l'inocultation
- La figure 2 :: représente la bioconversion de l'acide vanillique ajouté à 3 jours de culture.
- La figure 3 :: représente la bioconversion de l'acide vanillique en vanilline et alcool vanillique.
- La figure 4 :: représente la bioconversion de l'acide férulique ajouté dans de l'inoculation.
- La figure 5 :: représente la bioconversion de l'acide férulique ajouté à 3 jours de culture.
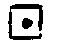 acide vanillique ou acide férulique respectivement
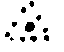 alcool vanillique
× vanilline
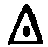 biomasse
Ac. Vanillique ou A.V. = acide vanillique
Alc. V. = alcool vanillique

### I - PROTOCOLE DE BIOCONVERSION

### 1) Principe

Le champignon filamenteux Basidiomycète est cultivé préalablement en milieu liquide agité afin d'obtenir une biomasse importante possédant les capacités enzymatiques nécessaires à une bioconversion ainsi qu'un rendement suffisant. Au bout de quelques jours de culture, le précurseur stérile est rajouté aseptiquement. La disparition du précurseur (acide férulique ou acide vanillique) et la bioconversion en alcool vanillique et en acide vanillique (pour l'acide férulique) sont suivies par analyse H.P.L.C.

### 2) Souches utilisées

Deux basidiomycètes de l'espèce Pycnoporus cinnabarinus déposée à la Collection Nationale de Culture de Microorganisme de l'Institut Pasteur (CNCM) sous les n° I-937 et I-938 le 10 Avril 1990.

### 3) Obtention de l'inoculum

Troi types d'inoculum sont possibles : il s'agit respectivement de spores ou de fragments de mycélium ou de leur préculture.

### a) Obtention de spores

Dans une fiole de Roux, 250 ml de milieu de sporulation (composition donnée en annexe 1) sont inoculés avec des fragments de mycélium de culture sur un milieu gélosé. L'incubation est réalisée à 37°C, pendant trois semaines. Pendant cette période, le mycélium se développe sur la surface gélosée, en produisant des conidies (spores). Ces spores sont décrochées de la surface de la culture solide par agitation de billes de verre baignant dans de l'eau physiologique stérile. La suspension de spores ainsi obtenue est filtrée sur de la laine de verre, afin d'éliminer tous fragments mycéliens de grande taille. Un dénombrement est réalisé sur une cellule de Thoma. On peut alors quantifier l'inoculum des cultures en utilisant un aliquot de volume connu de cette solution.

| Annexe 1 : Composition du milieu solide sporulation | |
|---|---|
| Extrait de levure | 3 g/l |
| Extrait de malt | 3 g/l |
| Bacto peptone | 5 g/l |
| Glucose | 10 g/l |
| Agar | 15 g/l |
| Biotine 0,5 ml/l d'une solution stérile à 0,001 % | |
| Solution de sels (1) | 20 ml/l |

| (1) Solution de sels | |
|---|---|
| Citrate-Na₃, 2H₂O | 125 g/l |
| kH₂PO₄ (anhydre) | 250 g/l |
| NH₄NO₃ (anhydre) | 100 g/l |
| MgSO₄, 7H₂O | 10 g/l |
| Solution d'oligoéléments (2) | 5 ml/l |

| (2) Solution d'oligoéléments | |
|---|---|
| Ac. citrique, H₂O | 50 g/l |
| Sulfate de zinc | 50 g/l |
| Sulfate de cuivre | 2,5 g/l |
| Sulfate de manganèse | 0,5 g/l |
| Ac. borique | 0,5 g/l |
| Na₂MoO₄, 2H₂O | 0,5 g/l |
| Fe(NH₄)₂(SO₄)₂, 6 H₂O | 10 g/l |

### b) Obtention de fragments de mycélium

Un milieu solide (20 g/l d'extrait de malt + 15 g/l d'agar + 2 g/l d'extrait de levure), en boîte de Petri, est inoculé centralement par un fragment de mycélium de culture sur milieu gélosé. L'incubation se fait à 37°C, jusqu'à ce que le mycélium recouvre totalement la surface. Des fragments de ce mycélium sont alors utilisés comme inoculum ; les fragments de mycélium sont découpés à l'emporte pièce d'un diamètre d'environ 4 mm.

### c) Obtention de préculture de mycélium

Une incubation des spores ou de fragments de mycélium est réalisée dans un volume de milieu (5 à 10 % du volume final de la culture) et ensuite utilisée comme inoculum.

### 4) Réalisation des cultures

100 ml de milieu comportant classiquement notamment une source de carbone animale ou végétale, des sels minéraux et des vitamines (une composition détaillée du milieu de culture utilisé est donnée en annexe 2) sont utilisés dans un Erlenmeyer de 500 ml de capacité. On inocule, soit par des fragments de mycélium (15 à 30 par culture), soit par des spores à raison de 2.10⁵ spores/ml de milieu. Les cultures sont incubées à 37°C, soumises à une agitation rotative de 120 tours/minute et d'amplitude 6 cm.

| Annexe 2 : Composition du milieu de culture | |
|---|---|
| Maltose | 20 g/l |
| Tartrate diammonium | 1,8415 g/l |
| KH₂PO₄ | 0,2 g/l |
| CaCL₂, 2H₂O | 0,0132 g/l |
| MgSO₄, 7H₂O | 0,5 g/l |
| Extrait de levure | 0,5 g/l |
| Chlorhydrate de thiamine | 2,5 mg/l |
| Stérilisation par autoclavage : | 120°C - 20 min. |

### 5) Supplémentation des cultures en précurseur

Après 72 h de culture, l'acide férulique ou l'acide vanillique stériles sont ajoutés, à raison de 0,3 g/l de culture. L'acide férulique et l'acide vanillique sont utilisés :
- soit sous forme de cristaux, autoclavés,
- soit sous forme de sel dans l'eau (par exemple, le sel de Na⁺, K⁺ ou NH⁺₄) et stérilisés par filtration (porosité : 0,2 µm).

### 6) Suivi cinétique de la conversion d'une culture

### a) Echantillonage

Chaque jour, dès la supplémentation en précurseur, on prélève stérilement 1,5 ml de chaque culture. Cet aliquot est filtré sur une membrane hydrophobe (porosité : 0,2 µm) prétraitée au méthanol et une analyse H.P.L.C. du filtrat est réalisée.

### b) Analyse H.P.L.C.

- phase inverse : colonne bondapack^{®} C18
- solvant : CH₃COOH 0,01 % dans H₂O/méthanol
- détection en UV à 280 nm
- volume d'échantillon injecté : 10 à 150 µl

### Temps de rétention des composés analysés

- alcool vanillique : 10 min,
- acide vanillique : 14 min,
- vanilline : 16 min,
- acide férulique : 18 min.

### 7) Mesure de la biomasse ("fausse cinétique")

Cette mesure nécessite le "sacrifice" de la culture ; les résultats obtenus dans ces conditions ne correspondent pas à une vraie cinétique, puisque ce n'est pas la même culture qui est suivie tout au long de l'étude.

Les cultures de 100 ml sont filtrées sur une membrane en fibres de verre, préalablement tarée. La biomasse receuillie est placée 24 h à 100°C. On détermine la matière sèche correspondante par pesée.

### II - RESULTATS

Exemple sur la souche n° I-937 (les mêmes résultats ont été obtenus avec la souche n° I-938).

### 1) Bioconversion d'acide vanillique

### a) Résultats avec mesure de la biomasse ("fausse cinétique")

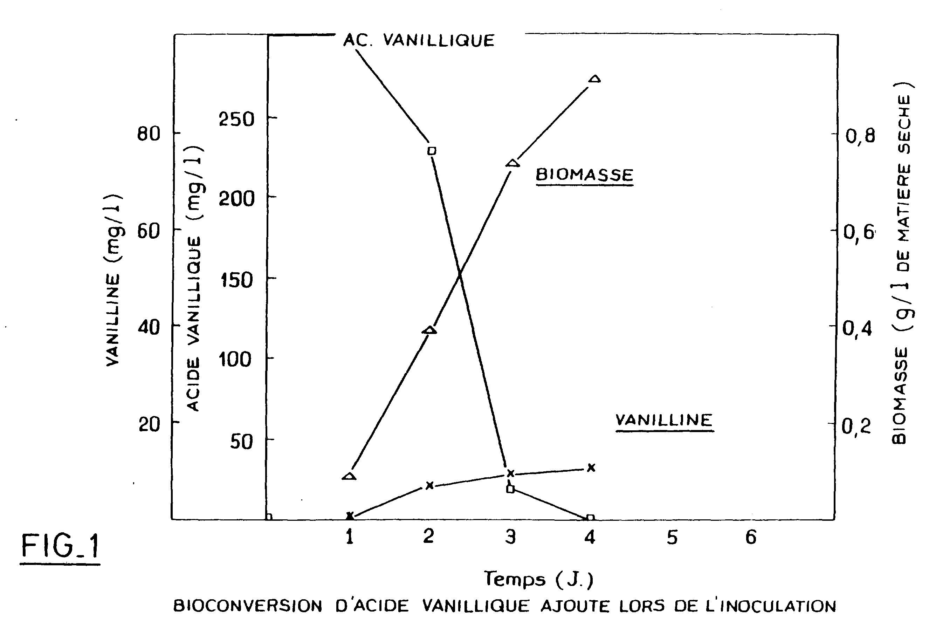

### PRECURSEUR AJOUTE LORS DE L'INOCULATION (voir figure 1)

| Age de la culture (en j) | Biomasse (en g de matière sèche par litre) | Précurseur acide vanillique (en mg/l) | Vanilline (en mg/l) |
|---|---|---|---|
| 0 | | 310 | |
| 1 | 0,088 | 314 | |
| 2 | 0,389 | 228 | 6,8 |
| 3 | 0,737 | 18 | 9,2 |
| 4 | 0,911 | 0,3 | 10,7 |

On observe une consommation rapide et totale de l'acide vanillique en 3-4 jours de culture. La biomasse microbienne augmente jusqu'au dernier jour d'analyse. La synthèse de vanilline est détectée dès le deuxième jour et la quantité de vanilline accumulée est croissante jusqu'au dernier jour de l'étude. Le rendement molaire maximal de conversion en vanilline est faible : 3,8 %.

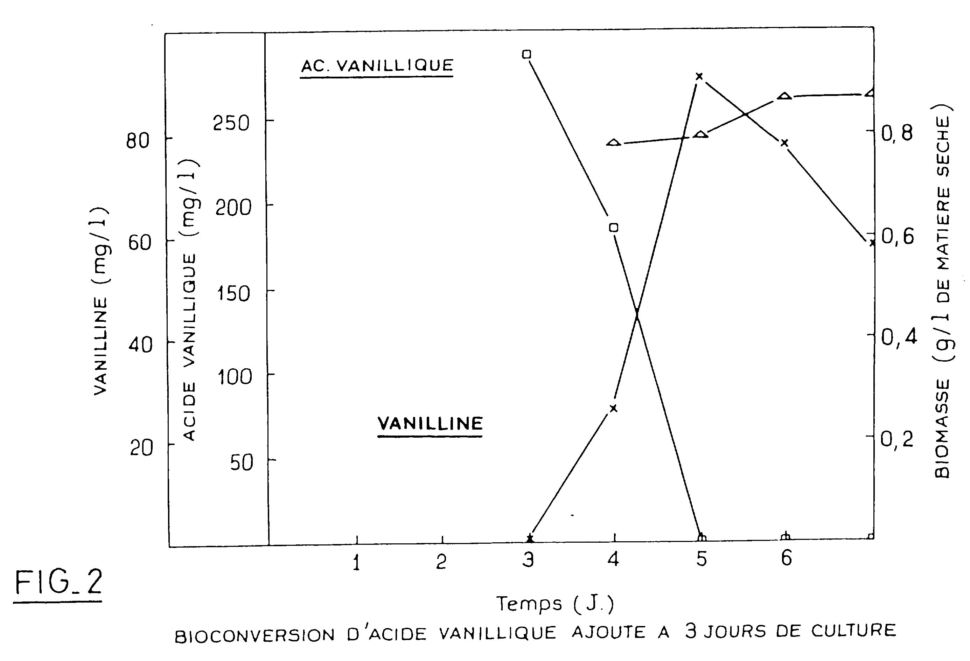

### PRECURSEUR AJOUTE AU 3EME JOUR DE CULTURE (voir figure 2)

| Age de la culture (en j) | Biomasse (en g de matière sèche par litre) | Précurseur acide vanillique (en mg/l) | Vanilline (en mg/l) |
|---|---|---|---|
| 3 | | 286 | |
| 4 | 0,782 | 184,5 | 25,6 |
| 5 | 0,798 | 0 | 90,8 |
| 6 | 0,869 | 0 | 78 |
| 7 | 0,872 | 0 | 58 |

La consommation d'acide vanillique est plus rapide que dans le cas précédent : en 2 jours la concentration en précurseur atteint 0 mg/l. La biomasse microbienne augmente peu au delà de la date d'apport en acide vanillique. La synthèse de vanilline est quasi immédiate et on atteint une teneur maximale 2 jours après la supplémentation. Au delà, la vanilline disparaît du milieu très rapidement. Dans ce cas, le rendement molaire maximal de conversion en vanilline est beaucoup plus élevé que précédemment : 35,1 %.

### b) Résultats en cinétique vraie, avec dosage de l'alcool vanillique

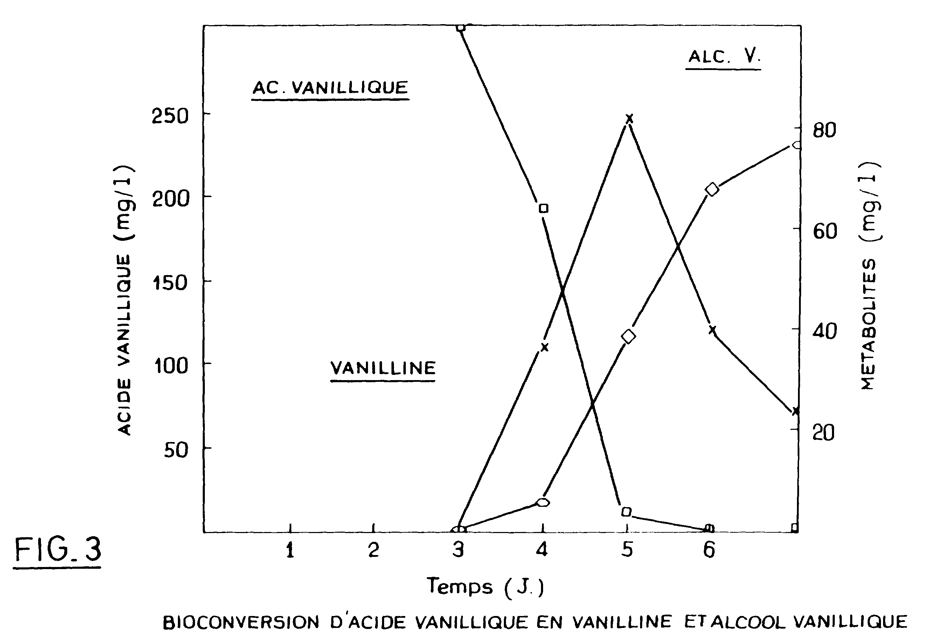

### PRECURSEUR AJOUTE AU 3EME JOUR DE CULTURE (voir figure 3)

| Age de la culture (en j) | Précurseur acide vanillique (en mg/l) | Vanilline (en mg/l) | Alcool vanillique (en mg/l) |
|---|---|---|---|
| 3 | 300 | | |
| 4 | 191 | 35,7 | 5,1 |
| 5 | 10,3 | 81,4 | 38,9 |
| 6 | 1,2 | 39,4 | 68,2 |
| 7 | 2,1 | 23,4 | 76,5 |

Les conditions de bioconversion sont les mêmes que précédemment ; les concentrations d'acide vanillique et de vanilline sont alors similaires ; la concentration en vanilline est maximale lorsque le concentration en précurseur atteint 0 mg/l. Les résultats de dosage de l'alcool vanillique donnent à penser que la vanilline est convertie en son alcool. Effectivement, l'apparition de l'alcool est décalée de 24 h environ par rapport à celle de la vanilline et sa concentration augmente considérablement lorsque la vanilline disparaît du milieu de culture. Un des moyens d'optimisation de la production serait de bloquer cette réduction, qui conduit à la formation d'alcool vanillique au détriment de la vanilline. Le rendement molaire maximal de conversion en vanilline est comparable au précédent : 31 %.

### 2) Bioconversion d'acide férulique

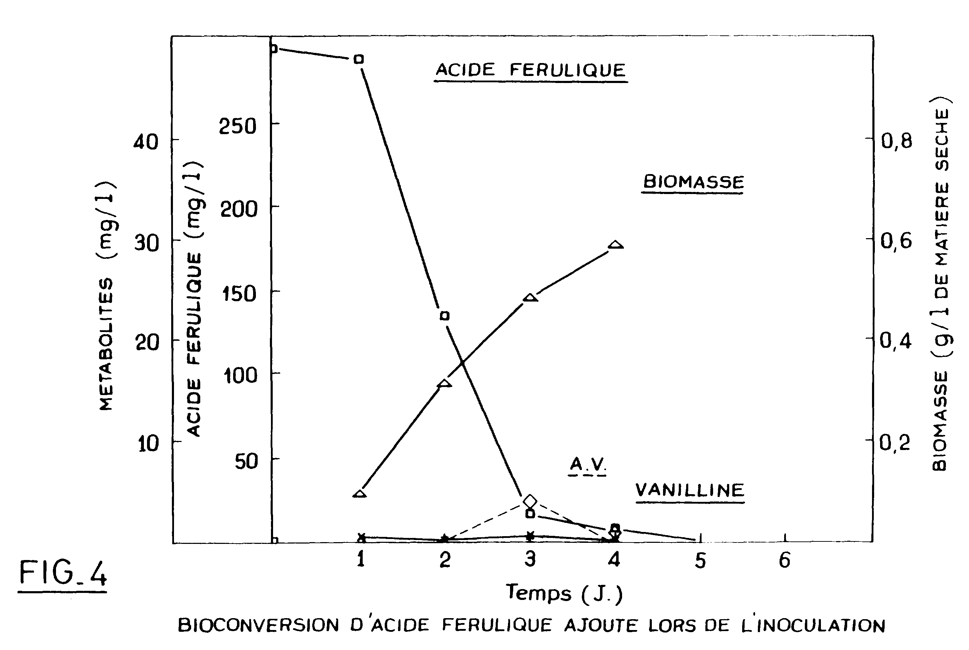

### PRECURSEUR AJOUTE LORS DE L'INOCULATION (voir figure 4)

| Age de la culture | Biomasse (en g de matière sèche par l) | Précurseur acide férulique (en mg/l) | Acide vanillique (en mg/l) | Vanilline (en mg/l) |
|---|---|---|---|---|
| 0 | | 294 | | |
| 1 | 0,095 | 287 | | |
| 2 | 0,314 | 133,7 | | |
| 3 | 0,484 | 15,7 | 4,2 | 0,5 |
| 4 | 0,590 | 7,4 | 1,1 | 0,35 |

Comme dans le cas de l'acide vanillique ajouté lors de l'inoculation, la biomasse augmente considérablement jusqu'au dernier jour de l'étude. La dégradation de l'acide férulique est totale au bout de 4-5 jours. On observe une synthèse d'acide vanillique et de vanilline, mais les rendements sont assez faibles (rendement molaire maximal de conversion en vanilline : 0,2 %).

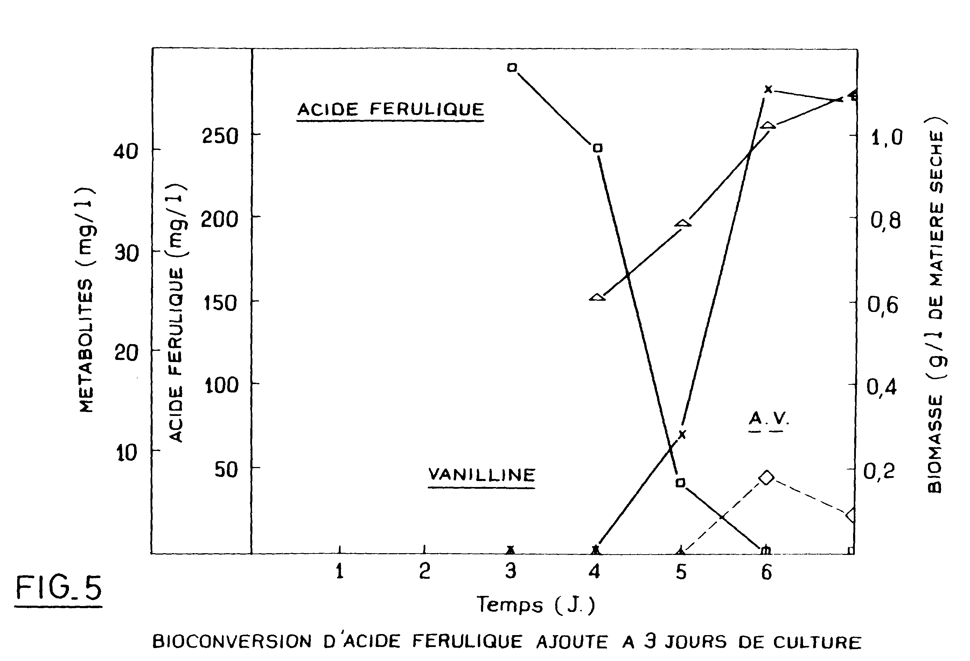

### PRECURSEUR AJOUTE AU 3EME JOUR DE CULTURE (voir figure 5)

| Age de la culture (en j) | Biomasse (en g de matière sèche par l | Précurseur acide férulique (en mg/l) | Acide vanillique (en mg/l) | Vanilline (en mg/l) |
|---|---|---|---|---|
| 3 | | 289 | | |
| 4 | 0,609 | 241 | | |
| 5 | 0,789 | 41,5 | | 11,5 |
| 6 | 1,023 | 0,9 | 7,7 | 46,2 |
| 7 | 1,099 | 0,85 | 4,1 | 45,4 |

Dans ce cas de supplémentation à 3 jours de culture, l'acide férulique disparaît totalement du milieu en 3 jours. On observe les synthèses simultanées d'acide vanillique et de vanilline. Comme pour l'acide vanillique, la quantité de vanilline est maximale lorsque la concentration en précurseur s'annule. Le rendement molaire maximal de conversion en vanilline est de : 20,5 %.

## Revendications

1. Procédé de production de vanilline caractérisé en ce qu'on effectue une culture de champignon Basidiomycète du genre Pycnoporus ou de leurs variants et mutants dans un milieu de culture dans lequel un précurseur benzènique de la vanilline a été ajouté et l'on récupère la vanilline produite par bioconversion dudit précurseur, la souche de pycnoporus étant sélectionnée pour sa propriété métabolique de dégrader lesdits précurseurs avec accumulation de vanilline dans le milieu.

2. Procédé selon la revendication 1, caractérisé en ce que le champignon est de l'espèce Pycnoporus cinnabarinus.

3. Procédé selon l'une des revendications 1 ou 2 caractérisé en ce que le précurseur provient de substrats végétaux naturels.

4. Procédé selon la revendication 3 caractérisé en ce que le précurseur benzènique est de l'acide férulique.

5. Procédé selon l'une des revendications précédentes, caractérisé en ce que le champignon est tout d'abord cultivé en milieu liquide agité, et l'on n'ajoute le précurseur qu'après qu'une biomasse importante possédant les capacités enzymatiques optimum pour la bioconversion dudit précurseur soit obtenue.

6. Procédé selon la revendication 5, caractérisé en ce que le précurseur n'est ajouté dans le milieu de culture qu'après au moins 3 jours de culture de Pycnoporus cinnabarinus.

7. Procédé selon l'une des revendications précédentes, caractérisé en ce que le champignon est inoculé dans le milieu de culture par un inoculum consistant en des fragments de mycelium ou en des spores, ou avec des précultures de ces inoculum.

8. Procédé selon l'une des revendications précédentes, caractérisé en ce que l'on récupère la vanilline lorsque le précurseur n'est plus détectable dans le milieu de culture et avant la bioconversion de la vanilline en alcool vanillique.

9. Procédé selon l'une des revendications précédentes, caractérisé en ce que l'on apporte le précurseur et l'on récupère la vanilline séquentiellement ou en continu.

10. Procédé selon l'une des revendications précédentes, caractérisé en ce qu'on utilise les souches de champignons Pycnoporus cinnabarinus CNCM n° I-937 et CNCM I-938 ou leurs variants et mutants possédant la propriété métabolique de dégrader les précurseurs benzéniques de la vanilline, tels que l'acide férulique avec accumulation de vanilline dans le milieu.

## Patentansprüche

1. Verfahren zur Herstellung von Vanillin, dadurch gekennzeichnet, daß man den Basidiomyceten-Pilz des Genus Pycnoporus oder Varianten und Mutanten davon in einem Kulturmedium kultiviert, dem ein Benzol-Vorläufer des Vanillins zugegeben worden ist, und das durch biologische Umwandlung aus dem Vorläufer gebildete Vanillin gewinnt (abtrennt), wobei der Pycnoporus-Stamm ausgewählt wird wegen seiner metabolischen Eigenschaft, die genannten Vorläufer abzubauen, unter Anreicherung von Vanillin in dem Medium.

2. Verfahren nach Anspruch 1, dadurch gekennzeichnet, daß es sich bei dem Pilz um die Species Pycnopors cinnabarinus handelt.

3. Verfahren nach den Ansprüchen 1 oder 2, daß der Vorläufer aus natürlichen Pflanzensubstraten stammt.

4. Verfahren nach Anspruch 3, dadurch gekennzeichnet, daß der Benzol-vorläufer die Ferulasäure ist.

5. Verfahren nach einem der vorhergehenden Ansprüche, dadurch gekennzeichnet, daß der Pilz zunächst in einem gerührten flüssigen Medium kultiviert wird und daß man den Vorläufer erst zugibt, nachdem eine beträchtliche Biomasse erhalten worden ist, die für die biologische Umwandlung des Vorläufers optimale enzymatische Fähigkeiten besitzt.

6. Verfahren nach Anspruch 5, dadurch gekennzeichnet, daß der Vorläufer dem Kulturmedium erst nach mindestens dreitägiger Kultivierung von Pycnopors cinnabarinus zugegeben wrid.

7. Verfahren nach einem der vorhergehenden Ansprüche, dadurch gekennzeichnet, daß der Pilz in das Kulturmedium inokuliert wird durch ein Inokulum, das besteht aus Mycelfragmenten oder aus Sporen oder mit Vorkulturen dieser Inokula.

8. Verfahren nach einem der vorhergehenden Ansprüche, dadurch gekennzeichnet, daß man das Vanillin gewinnt (abtrennt), wenn der Vorläufer in dem Kulturmedium nicht mehr nachweisbar ist und vor der biologischen Umwandlung des Vanillins in Vanillylalkohol.

9. Verfahren nach einem der vorhergehenden Ansprüche, dadurch gekennzeichnet, daß man die Zugabe des Vorläufers und die Gewinnung (Abtrennung) des Vanillins portionsweise oder kontinuierlich durchführt.

10. Verfahren nach einem der vorhergehenden Ansprüche, dadurch gekennzeichnet, daß man die Pilzstämme Pycnopors cinnabarinus CNCM Nr. I-937 und CNCM I-938 oder ihre Varianten und Mutanten verwendet, welche die metabolische Eigenschaft haben, die Benzol-Vorläufer des Vanillins wie Ferulasäure abzubauen unter Anreicherung von Vanillin in dem Medium.

## Claims

1. Process for producing vanillin, characterized in that culturing of a basidiomycete fungus of the genus Pycnoporus or of their variants and mutants is performed in a culture medium to which a benzenoid precursor of vanillin has been added, and the vanillin produced by bioconversion of said precursor is recovered, the Pycnoporus strain being selected for its metabolic property of degrading said precursors with accumulation of vanillin in the medium.

2. Process according to Claim 1, characterized in that the fungus is of the species Pycnoporus cinnabarinus.

3. Process according to either of Claims 1 and 2, characterized in that the precursor originates from natural plant substrates.

4. Process according to Claim 3, characterized in that the benzenoid precursor is ferulic acid.

5. Process according to one of the preceding claims, characterized in that the fungus is first cultured in an agitated liquid medium, and the precursor is added only after a large biomass, possessing optimal enzymatic capacity for bioconversion of said precursor, is obtained.

6. Process according to Claim 5, characterized in that the precursor is added to the culture medium only after at least 3 days of culture of Pycnoporus cinnabarinus.

7. Process according to one of the preceding claims, characterized in that the fungus is inoculated into the culture medium via an inoculum consisting of mycelium fragments or spores, or with precultures of these inocula.

8. Process according to one of the preceding claims, characterized in that the vanillin is recovered when the precursor is no longer detectable in the culture medium and before bioconversion of the vanillin to vanillic alcohol.

9. Process according to one of the preceding claims, characterized in that the precursor is introduced and the vanillin is recovered sequentially or in continuous fashion.

10. Process according to one of the preceding claims, characterized in that the strains of Pycnoporus cinnabarinus fungi CNCM No. I-937 and CNCM No. I-938 or their variants and mutants possessing the metabolic property of degrading benzenoid precursors of vanillin such as ferulic acid, with accumulation of vanillin in the medium, are used.
